# EUROPÄISCHE PATENTSCHRIFT

(11) **EP 0 912 891 B1**
(45) Veröffentlichungstag und Bekanntmachung des Hinweises auf die Patenterteilung: **16.10.2002**
(21) Anmeldenummer: 97923007.5
(22) Anmeldetag: 07.05.1997
(51) Int. Cl.: G01N 33/50, G01N 33/86, G01N 33/569, G01N 33/80

(54) **VERFAHREN ZUM DURCHFÜHREN VON BLUTTESTS**
METHOD OF CARRYING OUT BLOOD TESTS
PROCEDE POUR EFFECTUER UN EXAMEN DE SANG

(30) Priorität: 21.05.1996 DE 19620443
(43) Veröffentlichungstag der Anmeldung: 06.05.1999
(73) Patentinhaber: RHEIN BIOTECH GESELLSCHAFT FÜR NEUE BIOTECHNOLOGISCHE PROZESSE UND PRODUKTE MBH, 40595 Düsseldorf (DE); Menssen, Hans Dietrich, 14193 Berlin (DE); Thiel, Eckard, 14163 Berlin (DE)
(72) Erfinder: STRASSER, Alexander, W.M., D-40223 Düsseldorf (DE); MELBER, Karl, D-40591 Düsseldorf (DE); MENSSEN, Hans Dietrich, D-14193 Berlin (DE); THIEL, Eckard, D-14163 Berlin (DE)
(74) Vertreter: Grünecker, Kinkeldey, Stockmair & Schwanhäusser Anwaltssozietät
(86) Internationale Anmeldenummer: EP9702343
(87) Internationale Veröffentlichungsnummer: WO97044661

(56) Entgegenhaltungen:
- EP-A- 0 442 843
- WO-A-92/14150
- WO-A-96/10749
- DE-A- 4 329 969
- DATABASE WPI Section Ch, Week 9242 Derwent Publications Ltd., London, GB; Class A96, AN 92-345143 XP002041182 & JP 04 249 766 A (SEKISUI CHEM IND CO LTD) , 4.September 1992
- P. WLASMANN ET AL.: "Über den Einsatz des spezifischen Thrombininhibitors Hirudin für diagnostische und biochemische Untersuchungen." DIE PHARMAZIE, Bd. 43, November 1988, Seiten 737-744, XP002041181 in der Anmeldung erwähnt

## Beschreibung

Die Erfindung betrifft ein Verfahren zum Durchführen von Bluttests.

Zur Diagnosestellung insbesondere internistischer Erkrankungen sowie zu deren Verlaufskontrolle sind vielfältige Blutuntersuchungen erforderlich. Diese lassen sich einteilen in Untersuchungen der zellulären Komponenten des Blutes (Blutbild, Differentialblutbild, Blutgruppe, Immunphänotypisierung der Blutzellen) und Untersuchungen des Serums sowie Antikörpersuchteste, Coombsteste und Kreuzungsuntersuchungen. Bei den Untersuchungen des Serums handelt es sich um Bestimmungen von Enzymen, Stoffwechselprodukten (z.B. Kreatinin, Harnstoff, Blutzucker) und Gerinnungsparametern. Daneben werden aus dem Serum Spezialuntersuchungen wie z.B. Hormonbestimmungen oder Medikamentenspiegelbestimmungen durchgeführt.Beispielsweise wird in WO 96/10749 ein Testverfahren beschrieben, mit dem ermittelt werden kann, inwieweit der Blutplättchen-GPIIb/IIIa-Rezeptor eines Patienten mit Thromboserisiko blockiert ist.

Bei schwerkranken hospitalisierten erwachsenen Patienten sind über einen längeren Zeitraum täglich und z. T. sogar zweimal täglich erweiterte Blutuntersuchungen durchzuführen, um kritische Veränderungen des Gesundheitszustands der Patienten frühzeitig erfassen zu können. Die Blutabnahme erfolgt unter sterilen Bedingungen und in der Regel unter Verwendung eines geschlossenen Blutabnahmesystems durch Punktion einer Vene in der Armbeuge (z.B. V. cubita mediana). Gegebenenfalls wird das Blut über bereits liegende zentrale Verweilkatheter gewonnen. Je nach den klinischen Erfordernissen und den gewünschten Laboruntersuchungen wird die Art und die Anzahl der mit Blut zu füllenden Röhrchen festgesetzt. Bestimmte Laboruntersuchungen lassen sich nur aus speziell dafür hergerichteten und bestimmte Substanzen (z. B. bestimmte Antikoagulanzien (Heparin, EDTA, Citrat etc.) oder Glucosidaseinhibitor ("Blutzuckerröhrchen") oder gerinnungsfördernde Substanzen ("Serumröhrchen") enthaltenden Blutentnahmeröhrchen durchführen. Heparin und EDTA sind unspezifische, indirekte Inhibitoren. Sie wirken nicht direkt oder spezifisch auf ein bestimmtes Element der Blutgerinnungskaskade, sondern ihre Wirkung ist indirekter Natur, indem sie beispielsweise Ca²⁺-Ionen abfangen, die widerum für die Aktivität verschiedener Proteine des Gerinnungssystems essentiell sind. Ein Verfahren und ein Behälter zur Bestimmung der Funktionen und Zustandsänderungen von Blutzellen und Blutkorpuskeln mit einem sowohl Meizothrombin als auch Thrombin hemmenden, zweiwertige Kationen nicht-bindenden Inhibitor, wie z.B. Hirudin, werden in EP 0442843 A1 beschrieben.
Eine einheitliche Vorbehandlung des zu untersuchenden Blutes ("Standardröhrchen") aus dem dann alle oder zumindest die meisten wichtigen Blutuntersuchungen durchgeführt werden könnten, ist jedoch nicht verfügbar. Ferner ergibt sich aus dem üblichen logistischen Ablauf in klinischchemischen oder hämatologischen Laboratorien, daß häufig mehrere auf gleiche Weise vorbehandelte Blutabnahmeröhrchen angefordert werden. Nach der Abnahme vom Patienten werden die Blutröhrchen je nach zu bestimmenden Laborwerten in meist räumlich getrennte Laboratorien gebracht (meist ein klinisch-chemisches Labor und ein hämatologisches Labor und gegebenenfalls Labors für Spezialuntersuchungen wie Immunophänotypisierung, Medikamentenspiegel im Serum etc. (hier ist möglicherweise eine Materialversendung per Post oder Kurier erforderlich)). Die im Labor angekommenen Blutröhrchen müssen zunächst sortiert werden, die zu zentrifugierenden Röhrchen werden dann 5 Minuten bei ca. 3500 UpM zentrifugiert. Anschließend werden die Blutentnahmeröhrchen an die verschiedenen Arbeitsplätze weitergeleitet (Gerinnungsröhrchen an den Gerinnungsplatz, Serumröhrchen zur Elektrolytbestimmung an das Flammenfotometer etc.). Die entsprechenden automatischen Analysegeräte werden dann mit den Proben beschickt, wobei das für eine Messung erforderliche Probenvolumen mit ca. 10µl bis 100µl bei den meisten Meßvorgängen sehr gering ist. Der eigentliche Meßvorgang dauert nur Sekunden bis wenige Minuten (max. 5 Minuten, abhängig von der Methode und dem Gerät). Die Meßwerte werden schließlich ausgedruckt und je nach Herkunft den einsendenden Stationen schriftlich mitgeteilt.

Zusammenfassend ist es z.Z. erforderlich, daß bei einer Blutentnahme mehrere Blutentnahmeröhrchen (je nach gewünschter Untersuchung) mit 2-10 ml Blut (je nach Röhrchengröße) gefüllt werden müssen. Zur Bestimmung der Routine-Laborparameter sind dazu folgende Röhrchen erforderlich:

| | |
|---|---|
| Blutbild und Differentialblutbild | 1 x EDTA-Röhrchen |
| Natrium, Kalium im Serum | 1 x Serum-Röhrchen |
| Leberenzyme und/oder | |
| Kreatinin und/oder | |
| Harnstoff und/oder | |
| Lipase und Amylase und/oder | |
| Creatin-Kinase und/oder | |
| Cholesterin und Triglyceride und/oder | |
| Laktatdehydrogenase | 1 x Serum-Röhrchen |
| Gesamtprotein und Eiweißelektrophorese | 1 x Serum-Röhrchen |
| Glukose im Serum : | 1 x Glukose-Röhrchen |
| Quick, PTT | 1 x Gerinnungsröhrchen (Citrat) |
| Blutsenkungsgeschwindigkeit | 1 x BSG-Röhrchen |
| Gesamt | 7 Röhrchen |

Ein Blutentnahmegefäß mit Natriumcitrat und einem Thrombin-Inhibitor wie Hirudin, FUT oder (2R,4R)-4-Methyl-1-(N2-(3-methyl-1,2,3,4-tetrahydro-8-chinolin-sulphonyl)-L-alginyl)-2-piperidincarbonsäure-Monohydrat (MD805) wird in JP 04249766 A beschrieben.

Müssen dazu noch die Blutgruppe bestimmt werden, ein Antikörpersuchtest durchgeführt und Erythrozytenkonzentrate bereitgestellt werden, so sind weitere 2 Blutentnahmeröhrchen (ohne Zusätze) abzunehmen. Bei erforderlichen Spezialuntersuchungen wie Hormonspiegel-Bestimmungen (T3, T4, TSH basal etc.), Medikamentenspiegel (Digitoxin-Spiegel, Vancomycin-Spiegel, Theophyllin-Spiegel etc.), besondere Elektrolytkonzentrationen (Magnesium, Kalzium, Phosphat) und speziellen Gerinnungswerten (Faktoren-Mangel, Fibrin-Spaltprodukte) u. v. a., so sind in der Regel je ein zusätzliches Entnahmeröhrchen (meist Serum-Röhrchen) abzunehmen.

Bei diesen bisher allgemein üblichen Blutabnahmeverfahren ergeben sich somit unabhängig vom Blutabnahmesystem folgende gravierende Nachteile:
1. Die täglichen bei Schwerkranken erforderlichen Blutabnahmen führen zu einem Blutverlust von etwa 250 ml pro Woche. Dies entspricht etwa einer halben Blutspende eines Gesunden beim Deutschen Roten Kreuz. Erschwerend kommt hinzu, daß Schwerkranke häufig eine Blutarmut unterschiedlichster Ursache aufweisen.
2. Die vielen benötigten Blutentnahmen stellen einen erheblichen Kostenfaktor bei der medizinischen Versorgung dar, zum einen durch die Anschaffungskosten und zum anderen durch die erheblichen Entsorgungskosten der Röhrchen begründet. Die benutzten Blutröhrchen werden als infektiöser Naßmüll eingestuft und müssen in Spezialcontainern verpackt verbrannt werden. Bei Schwerkranken werden pro Woche etwa 40 Blutentnahmeröhrchen gebraucht.
3. Die durch die unterschiedlich vorbehandelten Blutproben (antikoaguliertes Vollblut für die Blutbildbestimmung, Serum für Enzymtestungen und Elektrolytbestimmungen etc.) und die darauf abgestimmten automatischen Meßgeräte festgelegte und segmentierte Arbeitsplatzeinteilung erfordert eine Vielzahl von Arbeitsplätzen mit entsprechend hohem personellen und finanziellen Aufwand.

Der vorliegenden Erfindung liegt die Aufgabe zugrunde, ein Verfahren zum Durchführen von Bluttests bereitzustellen, womit die vorstehend genannten Nachteile überwunden werden können, wobei rasch und zuverlässig in einer kurzen Zeitspanne zahlreiche Blutmeßparameter nahezu gleichzeitig bestimmt werden können.

Die Aufgabe wird durch ein automatisiertes Verfahren gelöst, bei dem eine frisch entnommene Blutprobe mit mindestens einem Thrombin-Inhibitor, ausgewählt aus Hirudin und/oder Desulfatohirudin, versetzt wird und diese Blutprobe sowohl für die Bestimmung klinisch-chemischer Parameter als auch für die Bestimmung hämatologischer Parameter eingesetzt wird. Der Lösung der Aufgabe liegt insbesondere die Erkenntnis zugrunde, daß nahezu alle klinisch-chemischen Blutparameter nicht nur unter Verwendung von Blutserum, sondern auch unter Verwendung von Blutplasma bestimmt werden können, sofern dieses Blutplasma entgegen der üblichen Praxis durch Zugabe eines Thrombin-Inhibitors, ausgewählt aus Hirudin und/oder Desulfatohirudin, bereitet wurde. Der Unterschied zwischen Serum und Plasma besteht darin, daß ersteres von Fibrin befreit ist, während letzteres noch Fibrinogen enthält. Überraschenderweise wurde gefunden, daß ein und dieselbe Blutprobe sowohl für die Bestimmung klinisch-chemischer Parameter als auch für die Bestimmung hämatologischer Parameter eingesetzt werden kann, wenn diese mit einem Thrombin-Inhibitor, ausgewählt aus Hirudin und/oder Desulfatohirudin, versetzt wird. Bevorzugt wird als Thrombin-Inhibitor rekombinantes Hirudin und/oder Desulfatohirudin eingesetzt.

Hirudin ist ein hochspezifischer Thrombin-Inhibitor, der natürlicherweise im Speicheldrüsensekret des Blutegels, Hirudo medicinalis, vorkommt. Die gerinnungshemmende Aktivität im Speicheldrüsensekret von Hirudo medicinalis wurde erstmals vor rund 100 Jahren durch Haycraft beschrieben (Haycraft, J.B. (1894), Naunyn-Schmiedebergs Arch. Exp. Pathol. Pharmakol. 18, 209). In den 50iger Jahren gelang es Markwardt et al. Hirudin in reiner Form zu gewinnen und biochemisch zu charakterisieren (Markwardt, F. und Walsmann, P. (1958), Hoppe-Seyler's Z. physiol. Chemie 312, 85). Es handelt sich dabei um ein Polypeptid, von dem inzwischen verschiedene natürlich auftretende Varianten bekannt sind, mit einem Molekulargewicht von ca. 7000 Dalton. Natürliches Hirudin wird als Thrombin-Inhibitor für biochemische Studien eingesetzt (Walsmann et al., (1988) Pharmazie 43, 737). Wegen der geringen im Blutegel vorkommenden Mengen und der aufwendigen Gewinnung des Hirudins beschränkte sich der Einsatz von Hirudin auf wenige sehr spezielle Anwendungen. Unter anderem wurde es auch zur Antikoagulation von Blutproben für eine Bestimmung der Funktion und Zustandsänderung von Blutzellen (z. B. Blutsenkungsgeschwindigkeit) vorgeschlagen (EP-A-442 843).

Seit Ende der 80iger Jahre ist es möglich, Desulfatohirudin durch gentechnologische Verfahren mittels Hefen oder Bakterien in großen Mengen herzustellen.Dieses rekombinante Desulfatohirudin ist (bis auf eine fehlende Sulfatgruppe am Tyrosin 63) mit dem natürlichen Hirudin aus Hirudo medicinalis identisch und besitzt die gleichen gerinnungshemmenden Eigenschaften. Durch die nach wie vor hohen Herstellungskosten wird rekombinantes Hirudin bevorzugt für einen Einsatz im therapeutischen Bereich entwickelt. Durch Einsatz neuartiger Expressionsysteme wie der Hefe Hansenula polymorpha konnte die Ausbeute jedoch derart gesteigert werden (Weydemann et al. (1995), Appl. Microbiol. Biotechnol 44,377), daß sich für Hirudin auch nicht therapeutische Anwendungen erschließen. Rekombinantes Desulfatohirudin ist leicht löslich und kann bereits in geringen Konzentrationen die Blutgerinnung hemmen. Dies ermöglicht es, rekombinantes Desulfatohirudin entweder in gelöster Form oder als Trockensubstanz vorzulegen und damit eine effektive Hemmung der Blutgerinnung zu erzielen. Die Menge an vorgelegtem Desulfatohirudin kann dabei so dosiert werden, daß das entnommene Blut je nach Zeiterfordernis der betreffenden Untersuchungen ungerinnbar bleibt. Weiterhin kann die Menge an Desulfatohirudin so bemessen werden, daß keine Verdünnungseffekte des abzunehmenden Blutvolumens entstehen und damit das Meßergebnis nicht beeinflußt wird. Die Einhaltung von Normvolumina ist kritisch bei Verwendung von Citrat-Lösung als Antikoagulans. Im Gegensatz zu Na-EDTA oder Citrat-Lösung beruht die antikoagulierende Wirkung bei Desulfatohirudin nicht auf dem Entzug von Ca²⁺-Ionen, sondern auf einer spezifischen sterischen Thrombininhibition. Dadurch werden dem Blut keine zweiwertigen Kationen entzogen, was eine Untersuchung von zellulären Blutkomponenten unter physiologischen Bedingungen erlaubt. Auch benötigt Desulfatohirudin für seine gerinnungshemmende Aktivität keine endogenen Faktoren wie dies zum Beispiel für Heparine der Fall ist. So benötigt Heparin für eine effiziente Hemmung der Gerinnung die Faktoren Antithrombin III und Heparin-Kofaktor 2. Mit Desulfato-Hirudin können Blutproben, die von Patienten mit einer entsprechenden Faktor-Defizienz stammen, ohne zusätzliche Maßnahmen problemlos analysiert werden. Aufgrund theoretischer Überlegungen ist zu erwarten, daß auch natürliche Hirudin-Varianten sowie Hirudin - Fragmente, soweit sie thrombininhibitorisch wirken, gleiche Erbebnisse liefern wie sie für rekombinantes Desulfatohirudin gefunden wurden. Weiterhin können auch synthetische ThrombinInhibitoren mit dem gleichen Ergebnis eingesetzt werden.

Bei der Untersuchung hämatologischer Parameter müssen die Blutzellen zum Zeitpunkt der Untersuchung vital sein. Das Blut muß antikoaguliert sein und darf in seinen Volumenanteilen durch den Untersuchungsgang nicht verändert werden, sonst treten Verfälschungen der Zahlenwerte auf. Hämatologische Untersuchungsparameter sind z.B. die Anzahl an Erythrozyten, Leukozyten und Thrombozyten pro Volumeneinheit Vollblut, die anteilsmäßige Zusammensetzung der Leukozyten aus den verschiedenen kernhaltigen Blutzellen, die nach morphologischen Kriterien bestimmt wird (Differentialblutbild), und der Grad der Beladung der einzelnen Erythrozyten mit Hämoglobin (Quotient aus dem klinisch-chemischen Meßwert Hämoglobinkonzentration und der Anzahl an einzelnen Erythrozyten pro Volumeneinheit). Weitere hämatologische Meßparameter beschreiben unter Anwendung immunologischer Testreagenzien die Oberflächenbeschaffenheit (Antigenität) intakter Erythrozyten (Transfusionsserologische Tests, Kreuzungen von Blutkonserven) oder mononukleärer Blutzellen (Immunphänotypisierung zur Diagnostik z.B. bösartiger Bluterkrankungen). Allen diesen hämatologischen Untersuchungen ist gemein, daß sie nur an unzerstörten, vitalen Blutzellen vorgenomen werden können.

Die Bestimmung hämatologischer Meßparameter erfolgt entweder manuell mikroskopisch (z.B. mittels Zählkammer zur Bestimmung der Anzahl an Blutzellen; Blutausstrichpräprat zur Beurteilung der Zellmorphologie (Zellgröße, Kernform, Zytoplasmaeigenschaften, zytoplasmatische Granulation, etc.)) oder in automatischen Blutzell-Zählgeräten (z.B. Coulter Counter). Die Bestimmung des Hämoglobinwertes im Blut ist ein klinisch-chemischer und kein hämatologischer Meßparameter.

Das vorliegende Verfahren beschreibt ein automatisiertes Verfahren, mit dem menschliches Vollblut in einem Blutentnahmeröhrchen in einer neuartigen Weise so antikoaguliert wird, daß diese Blutprobe simultan zur automatisierten Bestimmung von hämatologischen, klinisch-chemischen und immunologischen, kurzum zur Bestimmung fast aller Meßparameter eingesetzt wird. Dabei können die bisher üblichen Routine-Meßmethoden vollständig oder nach geringfügiger Adaptation an das neue Antikoagulans angewendet werden.

Ferner ist zu beachten, daß gemäß des vorliegenden Verfahrens eine quantitative Bestimmung mittels automatischer Meßgeräte erfolgt. Zur manuellen Bestimmung, wie bei der Zählung der Blutkörperchen zum Beispiel, wird frisches Kapillarblut verwendet. In größeren Laboratorien können hämatologische Untersuchungen heute aber nicht mehr manuell durchgeführt werden, sondern nur noch von automatisierten Meßgeräten. Zu diesem Zweck greift man auf venöses K2-EDTA-antikoaguliertes Vollblut zurück. Durch den Zusatz des Antikoagulans Dikalium-Ethylendiamintetraessigsäure (K2-EDTA) in der weltweit eingesetzten Standardkonzentration von 1 mg pro 1 ml Blut wird neben der erwünschten Komplexierung der Kalziumionen durch das EDTA auch eine hohe Konzentration an Kaliumionen in die Blutprobe gebracht. Diese artefiziell hohe Kaliumkonzentration bewirkt eine Veränderung des osmotischen Gradienten für Kalium zwischen den Leukozyten (Granulozyten und Lymphozyten) und dem umgebenden wäßrigen Mileu mit der Folge, daß die Leukozyten Zellwasser verlieren und schrumpfen. Nach einiger Zeit (30 Minuten) stellt sich durch Kompensationsmechanismen der Leukozyten ein neuer osmotischer Gradient für Kaliumionen ein und die Zellen gewinnen einen Teil des verlorenen Zellwassers zurück. Dieses neue Äquilibrium bleibt über mehrere Stunden stabil. Es ist natürlich anders im Vergleich zum nativen, nicht K2-EDTA-versetzten Blut. Erst wenn die kernhaltigen Blutzellen in K2-EDTA-antikoaguliertem Blut an dieses neue Äquilibrium adaptiert sind und eine geschrumpfte aber stabilisierte Form erreicht haben, sind sie in automatisierten Meßgeräten reproduzierbar auszählbar und sicher nach Volumen, Konduktivität und Streulichteigenschaften voneinander unterscheidbar (automatisierte Differntialblutbild-Erstellung). Ein der K2-EDTA Probenvorbehandlung ähnlicher Effekt wird mit Hirudin nicht beobachtet, was den Einsatz von Hirudinblut zur automatisierten Messung auszuschließen scheint. Die vorliegenden Ergebnisse zeigen jedoch überraschenderweise, daß mit Hirudin antikoaguliertem Blut quantitative hämatologische Meßparameter in automatisierten Meßgeräten bestimmt werden können.

Bei der automatisierten Bestimmung von hämatologischen Routineparametern, wie der Zahl der Erythrozyten, Leukozyten und Thrombozyten führen die Antikoagulanzien Natriumcitrat, Natriumoxalat und Heparin bekanntermaßen zu falschen Meßergebnissen. Der Zusatz von Natriumcitrat oder Natriumoxalat zu menschlichem Vollblut führt zu einer so erheblichen Schrumpfung der Erythrozyten, daß von deren automatisierter Größenbestimmung und den berechneten Folgewerten wie Hämatokrit nicht auf die Situation im nichtantikoagulierten nativen Blut geschlossen werden kann. Außerdem muß bei Verwendung von Natriumcitrat-antikoaguliertem Blut der Zahlenwert aller gemessenen Blutzellen sowie der Hämoglobinwert um den Faktor 1.1 korrigiert werden. Noch ungünstiger ist, daß sich dabei zufällige, bisher nicht befriedigend erklärbare Schwankungen der Zahlenwerte der Blutzellen finden, die bei den Thrombozyten eine Schwankungsbreite von 10%, bei pathologisch erniedrigten Thrombozytenwerten sogar darüber hinausgehend, ausmachen können. Durch die Verwendung von Heparin-antikoaguliertem Blut können unvorhersehbare, zufällige Spontanaggregationen von Thrombozyten und Leukozyten auftreten, so daß bei Auszählung in automatischen Meßgeräten z.T. falsch-niedrige Meßwerte für diese Zellen bestimmt werden. Erst recht treten Meßfehler durch die Verwendung von Antikoagulanzien, wie Heparin, Kalziumcitrat, aber auch K2-EDTA, bei der Bestimmung hämatologischer Parameter, auf, wenn die Leukozyten bei der automatischen Differenzierung im hydrodynamisch fokussierten Probenstrom nach Volumen (Widerstandsmessung im Gleichstrom), Konduktivität (Messung der internen Leitfähigkeit der Zellen mit hochfrequentem Wechselstrom) und Streulichteigenschaften (Messung der charakteristischen Oberflächenstrukturen der Zelle und deren randständiger Granulation mit einem Helium-Neon-Laser) differenziert werden (automatische Erstellung des Differentialblutbildes). So stört der Heparinzusatz bei der automatischen Erstellung des Differentialblutbildes insbesondere die exakte Erkennung der selteneren, aber diagnostisch besonders wichtigen basophilen Granulozyten. Typischerweise wird hierbei ein deutlich zu hoher Wert an basophilen Granulozyten angegeben. Außerdem kann ein solches fehlbestimmtes Blutbild aus dem Heparin-antikoagulierten Vollblut schlecht manuell nachbestimmt werden, was jedoch dringlich erforderlich ist. Der Heparinzusatz interferiert aufgrund seiner großen molekularen elektrostatischen Ladung mit den erforderlichen Färbelösungen (May-Grünwald-Lösung und Giemsa-Lösung), mit der die auf einem Glasobjektträger ausgestrichenen Blutzellen fixiert und angefärbt werden (panoptische Färbung nach Pappenheim). So kommt es durch Heparinzusatz zu einer Blaustichigkeit der kernhaltigen Blutzellen, die deren mikroskopische Differenzierung erschwert oder sogar unmöglich macht. Schließlich können sogar durch das zur Bestimmung von hämatologischen Routineparametern in automatisierten Meßgeräten aber auch bei manuellen Bestimmungen empfohlene und weltweit eingesetzte K2-EDTA gravierende Meßfehler auftreten. Durch Unterlassung von sofortigem Kippen und Bewegen der K2-EDTA-beschichteten Blutröhrchen nach der Blutentnahme kommt es häufig zur Bildung von kleinen Blutgerinnseln. Solcherart angeronnene Blutproben dürfen nicht verarbeitet werden, denn sie führen bei maschineller und manueller Bestimmung zu fehlerhaften Zahlenwerten der Blutzellen. Darüber hinaus können sie die Mikrokapillaren der automatischen Meßgeräte verstopfen.

In Anbetracht der Kenntnis dieser Vielzahl unerwünschter Interferenzen der unterschiedlichen Substanzen zur Antikoagulation menschlichen Vollblutes, die im einzelnen bei deren Einführung nicht vorhersehbar gewesen sind, war davon auszugehen, daß auch Hirudin-antikoaguliertes Vollblut mit einigen wichtigen Routinemeßverfahren ungünstig interferieren würde. Es bestand daher der generelle Vorbehalt in der Fachwelt, daß die Bestimmung einer Vielzahl klinisch-chemischer Parameter und hämatologischer Parameter aus einem einzigen Blutentnahmegefäß nicht möglich sei.

Mit dem erfindungsgemäßen Verfahren können jedoch aus einem einzigen Blutentnahmebehältnis gleichzeitig alle relevanten klinisch-chemischen sowie hämatologischen Werte ermittelt werden. Die möglichen Blutwertbestimmungen umfassen:
1. Bestimmung der Serumparameter (z.B. alkalische Phosphatase, Amylase, Cholinesterase, Kreatinkinase, GOT, GPT, γ-GT, HBDH, Lactat, LDH, Lipase, Albumin, Bilirubin, Calcium, Chlorid, Cholesterin, Kreatinin, Eisen, Gesamteiweiß, Glucose, Harnsäure, Harnstoff, Kalium, Magnesium, Natrium, Triglyceride, C-reaktives Protein, Immunglobuline, Transferrin, Anti-Streptolysin-O, Rheumafaktoren, C3, C4, Apolipoprotein, Medikamentenspiegel). Bevorzugt erfolgt die Bestimmung nach Abtrennen der korpuskulären Bestandteile aus Vollblut in automatisierten Meßgeräten. Zur weiteren Vereinfachung des Verfahrens kann die Bestimmung der Serumparameter auch in automatisierten Meßgeräten erfolgen, bei denen die Abtrennung der korpuskulären Bestandteile nicht mehr notwendig ist.
2. Untersuchung des Blutbildes: kleines Blutbild (automatisiert) und Differentialblutbild (manuell und automatisiert).
3. Bestimmung der Blutgruppe, Durchführung von Antikörpersuchtests, Durchführung von Coombstests und Auskreuzung von Erythrozytenkonzentraten.
4. Immunphänotypisierung normaler und maligner mononukleärer Zellen in Blut und Knochenmark.

Ein für die vorliegende Erfindung verwendbares Blutentnahmeröhrchen (universelles Standardröhrchen) kann ein Blutentnahmegefäß sein, das den Thrombin-Inhibitor, ausgewählt aus Hirudin und/oder Desulfatohirudin, als Lösung oder als Trockensubstanz oder als Oberflächenbeschichtung enthält. Die Menge des Thrombin-Inhibitors sollte dabei so bemessen werden, daß für eine Zeitdauer, in der alle oben beschriebenen Bestimmungen durchgeführt werden können, die Gerinnung des entnommenen Blutvolumens vollständig gehemmt wird und die problemlose Durchführung der Messungen im aufgezeigten Verfahren erlaubt. Als Blutentnahmegefäße eignen sich beispielsweise Röhrchen, Spritzen, Pipetten und Kapillaren aus Kunststoff, Glas oder Metall.

Eine weitere Ausführungsform der vorliegenden Erfindung bezieht sich auf die Durchführung des Bluttests mit automatisierten Meßgeräten, die folgende Funktionen in einer Einheit vereinigen:
- Bestimmung aller klinisch-chemischen Parameter
- Bestimmung aller hämatologischen Parameter,
wobei im Meßverlauf zuerst die hämatologischen Werte (Blutbild/Differentialblutbild) erfaßt und anschließend, nach wahlweiser Entfernung der zellulären Blutbestandteile (=Hirudin-Plasma), die klinisch-chemischen Parameter (einschließlich Spezialbestimmungen) ermittelt werden sollten. Die Entfernung der zellulären Bestandteile kann z. B. über ein Mikrofilter oder durch Zentrifugation erfolgen. Weiterhin können die oben beschriebenen Blutentnahmegefäße auch mit Meßgeräten verwendet werden, die Bestimmungen mittels Teststreifen durchführen.

Eine weitere Aufgabe der vorliegenden Erfindung ist es, ein Verfahren zum Bestimmen von klinisch-chemischen Blutparametern bereitzustellen, das einfacher und schneller durchführbar ist als bekannte Verfahren.

Die Aufgabe wird durch ein automatisiertes Verfahren gelöst, bei dem eine frisch entnommende Blutprobe mit mindestens einem Thrombin-Inhibitor, ausgewählt aus Hirudin und Desulfatohirudin, versetzt wird und die klinisch-chemischen Parameter unter Verwendung der so erhaltenen Blutprobe bestimmt werden. Es wurde überraschenderweise gefunden, daß eine frisch entnommene Blut- bzw. Knochenmarkprobe, die mit einem Thrombin-Inhibitor, ausgewählt aus Hirudin und/oder Desulfatohirudin, versetzt wird, zur Bestimmung der vorstehend genannten klinisch-chemischen Parameter verwendet werden kann. Eine Abtrennung des Fibrinogens ist nicht notwendig. Vorzugsweise können jedoch die zellulären und korpuskulären Bestandteile, wie vorstehend beschrieben, von dem so bereiteten Blutplasma abgetrennt werden.

Es ist vorteilhaft, als Thrombin-Inhibitor rekombinantes Hirudin und/oder Desulfatohirudin einzusetzen.

Auch bei diesem Verfahren sollte die frisch entnommene Blutprobe in ein Entnahmegefäß gegeben werden, worin der Thrombin-Inhibitor vorgelegt ist.

Die Bestimmung der klinisch-chemischen Parameter kann mittels eines automatisierten Meßgeräts erfolgen.

Die vorliegende Erfindung wird anhand der nachfolgenden Beispiele näher erläutert.

### Beispiele

1. Es konnte gezeigt werden, daß durch den Einsatz von rekombinantem Hirudin der Firma Rhein Biotech, Düsseldorf, menschliches Vollblut in Blutabnahmeröhrchen proportional zur eingesetzten Hirudinmenge unterschiedlich lange ungerinnbar gemacht werden konnte. Bei einer Konzentration von 200 ATU rHirudin (= 20 ng rHirudin) pro ml Vollblut war das Blut über 24 Stunden ungerinnbar, bei einer Konzentration von 100 ATU rHirudin (= 10 ng rHirudin) geronn Vollblut bereits nach 12 Stunden in Glas- oder Kunststoffabnahmeröhrchen vollständig (siehe Tabelle 1). 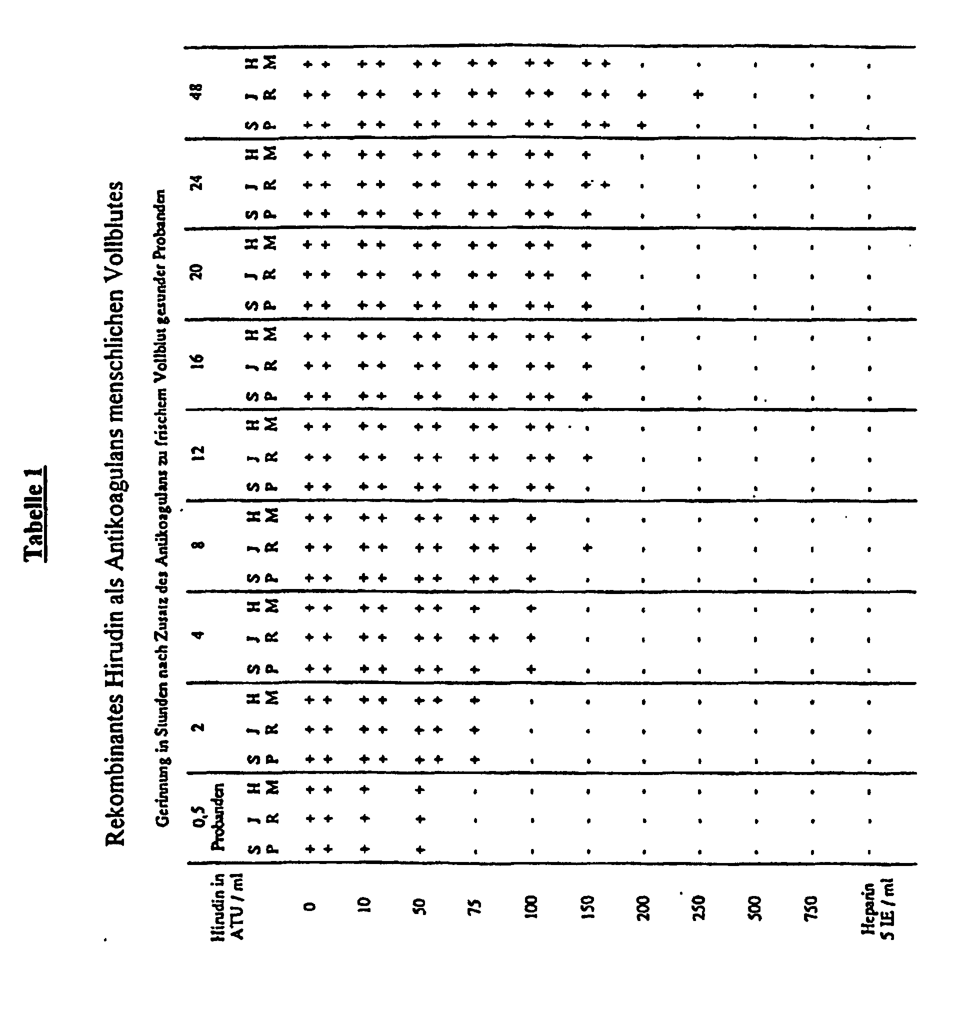
Jeweils 2 ml Vollblut von drei gesunden Probanden (SP, JR, HM) wurden unmittelbar nach Entnahme in nicht-präparierte Glasröhrchen des Vacutainer-Blutabnahmesystems (Fa. Becton and Dickinson) gegeben, die zuvor mit unterschiedlichen Mengen des rekombinanten Hirudin beschickt worden waren. Die Stammlösung des rekombinanten Hirudin betrug dabei 10⁴ ATU pro ml, so daß zwischen 20 und 150 µl Stammlösung pro Röhrchen eingesetzt wurden. Als positive Kontrolle wurde 5 IE/ml Heparin eingesetzt. Die Röhrchen wurden permanent auf einem Roller in Bewegung gehalten. Die Zeit bis zum Auftreten von kleinen ca. 1-2 mm durchmessenden Gerinseln (beginnende Gerinnung (+)) bzw. bis zu Auftreten einer kompletten Gerinnung (++) wurde gemessen. Das Vorhandensein von für den Zellzahl-Meßvorgang ungünstigen und das Ergebnis verfälschenden Mikrogerinseln in den Blutröhrchen wurde durch wiederholte Zellzählungen mittels des Gerätes STKS der Fa. Coulter geprüft. Der Nachweis von Mikrogerinseln wurde als beginnende Gerinnung gewertet (+). Waren kein Makro- oder Mikrogerinsel nachweisbar, so konnte das antikoagulierte Blut im STKS der Fa. Coulter problemlos analysiert werden (-).
2. Es konnte gezeigt werden, daß aus Glas-Blutabnahmeröhrchen, die mit 200 ATU rHirudin pro ml Blut beschickt worden waren, nach Zentrifugation der korpuskulären Elemente eine Vielzahl von klinisch-chemischen Laborbestimmungen an automatisierten Meßgeräten problemlos und reproduzierbar durchgeführt werden konnten. Die dabei erhobenen im pathologischen wie im Normbereich liegenden Meßwerte waren statistisch nicht signifikant unterschiedlich zu den mit der konventionellen Methode erhobenen Werten (Serummethode). Technische Probleme an den Meßgeräten, etwa Verstopfungen der Ansaugkapillaren etc. traten selten und nicht häufiger als mit der Serummethode auf. Dabei wurden folgende Meßparameter automatisiert bestimmt: GOT, GPT, Alkalische Phosphatase, gamma-GT, Natrium, Kalium, Kreatinin, Harnstoff, Kreatinkinase, Bilirubin, Lactatdehydrogenase, alpha-HBDH, Amylase, Lipase, Glucose, Gesamtcholesterin, Triglyceride, Chlorid, Magnesium, Phosphat, Kalzium, Eisen, Gesamtprotein, Eiweißelektrophorese, Antistreptolysintiter, C-reaktives Protein, beta-HCG (siehe Tabelle 2).

**Tabelle 2**

| Proband M Parameter | Serum | Hirudin-Plasma | Einheit | |
|---|---|---|---|---|
| GOT/ASAT | 9 | 9 | U/l | |
| GPT/ALAT | 10 | 10 | U/l | |
| AP | 103 | 100 | U/l | |
| GGT | 10 | 10 | U/l | |
| HBDH | 96 | 98 | U/l | |
| Bilirubin ges. | 12,7 | 13 | µmol/l | |
| CK | 60 | 59 | U/l | |
| Lipase | 79 | 82 | U/l | |
| LDH | 166 | 167 | U/l | |
| Amylase | 75 | 73 | U/l | |
| Kreatinin | 94 | 88 | µmol/l | |
| Harnstoff | 6,6 | 6,4 | mmol/l | |
| Ges. Chol. | 4,7 | 4,7 | mmol/l | |
| Triglyceride | 1,6 | 1,6 | mmol/l | |
| | | | | |
| Glucose | 5,3 | 5,5 | mmol/l | |
| | | | | |
| Caicium | 2,22 | 2,19 | mmol/l | |
| Magnesium | 0,7 | 0,73 | mmol/l | |
| Phosphat | 1,2 | 1,1 | mmol/l | |
| Eisen | 20 | 20,1 | µmol/l | |
| | | | | |
| Kalium | 4,2 | 3,8 | mmol/l | |
| Natrium | 142 | 141 | mmol/l | |
| Chlorid | 106 | 108 | mmol/l | |
| Osmolalität | 276 | 278 | mosmol/Kg | |
| | | | | |
| Ges. Protein | 76 | 77 | g/l | |
| Elektrophorese | | | | |
| Albumin | 70,6 | 67,3 | % | |
| Alpha 1 | 2,2 | 2,1 | % | |
| Alpha 2 | 5,3 | 5,8 | % | |
| Beta | 9,5 | 11,2 | % | |
| Gamma | 12,4 | 13,6 | % | mit Gradienten! |
| | | | | |
| AST | <80 | 120 | kU/l | |
| CRP | <6 | <6 | mg/l | |
| HCG | <2 | <2 | U/l | |

3. Es konnte gezeigt werden, daß aus dem gleichen Hirudinbehandelten Glas-Blutabnahmeröhrchen zusätzlich an automatischen Meßgeräten das kleine Blutbild (Bemessung und Berechnung der zellulären Anteile im Vollblut) sowie an automatischen Geräten wie auch per Hand das Differentialblutbild erstellt werden konnte. Die dabei erhobenen im Pathologischen wie im Normalen liegenden Meßwerte waren statistisch nicht signifikant unterschiedlich zu den mit der Routinemethode (EDTA-Blut) erstellten Werten. Technische Probleme an den automatisierten Zellzählgeräten (Fa. Coulter, USA) wie Verstopfung der Ansaugkanüle oder R-Alarm (Registrierfehler) traten dabei selten und nicht häufiger als mit der Standardmethode (EDTA-Blut) auf. Zur korrekten automatischen Bestimmung des Differentialblutbildes von rHirudin-antikoagulierten Vollblut müssen allerdings die Meßautomaten, die auf EDTA-antikoaguliertes Blut eingestellt sind, entsprechend für die Verwendung von rHirudin-antikoaguliertem Blut eingestellt werden. Insbesondere granulozytäre Zellen schrumpfen und verändern sich durch den EDTA-Zusatz in den ersten 60 Minuten. Dies führt dazu, daß die automatisierte Differentialblutbilderstellung von EDTA-Blut z.B. am Gerät STKS der Fa. Coulter erst dann verläßlich ist, wenn das Blut vor Untersuchung mindestens ca. 30 - 60 Minuten mit EDTA antikoaguliert war. Diese Maßnahme, die durch das EDTA-bedingte unspezifische Abfangen der freien Kalzium-Ionen notwendig ist, kann bei der die Zellen weniger beeinträchtigenden hochspezifischen Antikoagulation mittels rHirudin entfalten. Die Morphologie der kernhaltigen Zellen im handgezählten Differentialblutbild war in rHirudin-antikoaguliertem Blut besser erhalten, als im EDTA-Blut, insbesondere nach einer Zeit von mehreren Stunden.

### Folgender Versuch wurde durchgeführt:

Einem gesunden Probanden (XM) wurde venöses Blut aus der Cubitalvene entnommen und in Glas-Blutentnahmeröhrchen des Vacutainer-Blutabnahmesystems (Fa. Becton and Dickinson) gegeben. Diese vom Hersteller nicht vorpräparierten und zur Gewinnung von Serum bestimmten Röhrchen wurden zuvor mit Heparin (5 IE/ml) oder rekombinantem Hirudin (200 ATU/ml) beschickt. Als Kontrolle diente ein EDTA-Blutbildröhrchen (Fa. Becton and Dickinson), das ebenfalls mit venösem Nativblut des Probanden gefüllt wurde.

Vier Stunden nach der Blutentnahme (durchschnittliche Transport- und Versandzeit) wurden die Blutproben maschinell am STKS der Fa. Coulter ausgewertet. Das EDTA-Blut wurde zusätzlich sofort nach Entnahme ausgewertet. Dabei wurde das kleine Blutbild (Zahlenwerte) sowie das Differentialblutbild maschinell erstellt. Ferner wurden Ausstrichpräparate angelegt und daraus am folgenden Tag das Differentialbild manuell unter Mikroskop angefertigt.

Zusammenfassend konnten die Blutproben unabhängig vom eingesetzten Antikoagulans vergleichbar gut ausgewertet werden. Bei den Zahlenwerten im kleinen Blutbild und bei den manuell angefertigten Differentialblutbildern ergaben sich keine wesentlichen Unterschiede. Bei der maschinellen Anfertigung der Differentialblutbilder fand sich an dem auf EDTA-Blut kallibrierten Gerät STKS der Fa. Coulter jedoch sowohl bei Heparinblut als auch Hirudinblut erwartungsgemäß eine Fehlbemessung eines Teils der neutrophilen Granulozyten, die entweder als basophile Granulozyten oder als Monozyten erkannt wurden. Diese Fehlbemessung findet sich in gleicher und bekannter Weise, wenn EDTA-Blut innerhalb der ersten Stunde nach Blutabnahme maschinell ausgewertet wird. Durch entsprechende Kallibrierung könnte dieser Meßfehler korrigiert werden.
4. Es konnte darüber hinaus gezeigt werden, daß aus dem gleichen rHirudin-behandelten Glas-Blutentnahmeröhrchen zusätzlich zu den bereits beschriebenen Untersuchungen auch die Blutgruppe serologisch bestimmt, ein Antiköpersuchtest durchgeführt, die Coombs-Testungen durchgeführt und Austestungen von Erythrozytenkonzentraten gemacht werden konnten. Diese Untersuchungen konnten verläßlich und reproduzierbar durchgeführt werden. Technische Probleme traten dabei nicht auf. Hervorzuheben ist, daß gerade Antikörpersuchtests möglichst mit EDTA-freiem Blut durchgeführt werden sollten, um Interferenzen mit komplementabhängigen Antikörpern wie z.B. Kit a, Kit b, Lewis a und Lewis b möglichst zu vermeiden.
   Die Zukunft bei der Durchführung von Antikörpersuchtests und Blutkonservenkreuzungen, die z.Z. überwiegend manuell aus geronnenem Blut mittels der Tropftechnik und durch visuelles Ablesen der Agglutination erstellt werden, liegt insbesondere bei großen Blutbanken und Krankenhäusern in der Automatisierung des Pipettier- und Ablesevorgangs. Da den bei Auskreuzungen von Blutkonserven vom Patienten sowohl Serum oder Plasma als auch Erythrozyten gebraucht werden, muß das Patientenblut antikoaguliert und zentrifugiert sein, wenn die Kreuzung automatisiert mittels Pipettenautomat erfolgen soll. Ansonsten würden Blutkoagel bei der Entnahme der zentrifugierten Erythrozyten aspiriert werden und der Test müßte abgebrochen werden. Die Antikoagulation kann zwar mit EDTA erfolgen. EDTA-antikoaguliertes But eignet sich allerdings nur bedingt für die Durchführung von Antikörpersuchtests, da das EDTA mit Komplement interagiert und somit komplementabhängige Antikörper wie Kit a und b, Lewis a und b dann möglicherweise nicht mehr nachgewiesen werden können. Dagegen konnte erstmalig gezeigt werden, daß mit rHirudin antikoaguliertem Blut Antikörpersuchtests und Blutkreuzungen automatisiert mit Hilfe des Pipettierautomaten ID-Sampler II unter Verwendung des Micro Typing Systems (beide DiaMed AG, Cressier-sur-Morat, Schweiz) problemlos durchführbar waren.

### Folgende Versuche wurden durchgeführt:

### A (Vergleichsversuch) : Manuelle Blutgruppenbestimmung und manueller Antikörpersuchtest

5 ml venöses Vollblut eines gesunden Probanden wurden in ein unbehandeltes bzw. ein zuvor mit rekombinantem Hirudin (200 ATU/ml) beschicktes Glas-Blutentnahmeröhrchen (Fa. Becton and Dickinson) gegeben. Nach 30 Minuten war das Blut im unbehandelten Glasröhrchen vollständig geronnen, das Hirudin-behandelte Blut hingegen war antikoaguliert. Beide Glasröhrchen wurden nun zentrifugiert. Anschließend wurde aus beiden Glasröhrchen jeweils serologisch durch manuelle Tropftechnik und visuelles Ablesen der Agglutination die Blutgruppe bestimmt. In gleicher Weise wurden darüberhinaus Antikörpersuchtests mit einem Antikörper-Panel (Screening) in 3 verschiedenen Milieus (0,9% NaCl, Bromelin und Coombs) durchgeführt.

Zusammenfassend konnte rHirudin-antikoaguliertes Blut zur Blutgruppenbestimmung und zu Antikörpersuchtesten in gleicher Weise eingesetzt werden wie geronnenes Blut (Standard-Methode).

### B: Automatische Durchführung von Antikörpersuchtests mittels des Pipettierautomaten ID-Sampler II Micro-Typing Systems, Fa. DiaMed AG, Cressier sur-Morat, Schweiz.

5 ml venöses Vollblut eines gesunden Probanden wurden in ein zuvor mit rekombinantem Hirudin (200 ATU/ml) beschicktes Glas-Blutentnahmeröhrchen (Fa. Becton and Dickinson) gegeben. Das antikoagulierte Blut wurde im Glasröhrchen zentrifugiert. Das zentrifugierte Blutentnahmeröhrchen wurde anschließend in den Pipettierautomaten ID-Sampler II von DiaMed gegeben. In diesem Pipettierautomaten wurde dann Plasma aus dem Blutröhrchen entnommen und in die entsprechenden, zur Durchführung eines Antikörpersuchtests im NaCl-, Coombs- und Kälte-Milieu vorgefertigten Micro Typing Cards der Fa. DiaMed-ID pipettiert. Diese Micro Typing Cards wurden dann bei 37°C inkubiert und anschließend zentrifugiert. Zur exakten Identifizierung der Proben wurden dann die Barcodes auf den Micro Typing Cards manuell mittels eines Barcodelesers in einen Compacq 4/50 PC eingegeben. Schließlich wurden die Micro Typing Cards in den ID Reader M von DiaMed gestellt, die Agglutination photooptisch ausgewertet und über den Computer ausgedruckt.

Zusammenfassend konnte gezeigt werden, daß rHirudin-antikoaguliertes Vollblut im Pipettierautomaten ID-Sampler II Micro Typing Systems der Fa. DiaMed problemlos zur automatischen Durchführung von Antikörpersuchtesten eingesetzt werden kann.
5. Schließlich konnte gezeigt werden, daß sich der Immunphänotyp normaler und maligner (Leukämie, Lymphom) mononukleärer Zellen im menschlichen Blut oder Knochenmark nicht verändert, wenn das Untersuchungsmaterial nicht wie üblich durch Heparinzusatz sondern durch Zugabe von rHirudin antikoaguliert wird. Die Zellmorphologie im rHirudin-antikoagulierten Blut oder Knochenmark war dabei auch noch nach 96 Stunden Liegezeit bei Raumtemperatur (Simulation eines Versandweges) deutlich besser als in Heparinblut (Standardmethode).

### Folgender Versuch wurde durchgeführt:

Zur Immunphänotypisierung von Knochenmarkblut (A) oder peripherem Blut (B) wurde jeweils 5 ml Material in eine mit Heparin (5 IE/ml) oder rekobminantem Hirudin (200 ATU/ml) vorpräparierte Kunststoffspritze aspiriert. Der Patient A ist an einem Rektumkarzinom erkrankt. Die Untersuchung seines Knochenmarks war wegen des Verdachtes auf das zusätzliche Vorliegen einer akuten myeloischen Leukämie durchgeführt worden. Die Patientin B ist an einer chronischen myeloischen Leukämie in chronischer Phase erkrankt. Die Untersuchung aus dem peripheren Blut war zur Diagnoseabsicherung durchgeführt worden. Eine Stunde (A) bzw. drei Tage (B, Simulierung des Transportweges bei Versandmaterial, eine häufige Konstellation) nach Antikoagulation bei Raumtemperatur wurde das Material über einen Ficoll-Gradienten (Fa. Pharmacia) zentrifugiert und die mononukleären Zellen somit separiert. Die mononukleären Zellen wurden mit einer Pipette in ein Glasröhrchen überführt, die Zellzahl am Coulter Counter bestimmt und auf eine Zellzahl von 5x 10⁵ mit 0,9% NaCl eingestellt. Zu Fraktionen dieser Zellsuspension wurden dann verschiedene primäre Antikörper pipettiert. Nach Inkubation und Waschen der Zellsuspension wurde ein sekundärer Fluoreszenz-markierter Antikörper (FITC-konjugierter F (ab')₂ Ziegen anti-Maus) zur Detektion des zellmembran-gebundenen primären Antikörpers eingesetzt (indirekte Immunfluoreszenz). Die mit Antikörpern behandelten Zellsuspensionen wurden nun durchflußzytometrisch analysiert (FACScan®, Fa. Becton und Dickinson). Die Zahlenwerte entsprechen dem Prozentsatz der fluoreszierenden Zellen, die ausgewertet wurden.
Zusammenfassend zeigt sich, daß sowohl Heparin-antikoaguliertes Blut (Standardmethode) als auch Hirudin-antikoaguliertes Blut sofort und nach 3 Tagen vergleichbar gut immunphänotypisch mittels FACScan® analysiert werden kann und daß diese Untersuchung unabhängig von der Antikoagulation zu vergleichbaren Ergebnissen führt.

Zusammenfassend konnte erstmalig gezeigt werden, daß mit rHirudin-antikoaguliertem Blut aus einem Blutentnahmeröhrchen sowohl eine Vielzahl von klinisch-chemischen Routineund Spezialuntersuchungen (Verwendung des Plasmas) als auch blutgruppenserologische, zytomorphologische und quantitative Blutzellbestimmungen (Verwendung des Vollblutes) gleichwertig zu den eingeführten Routinemethoden möglich sind. Daraus sind im Praktischen folgende Verbesserungsmöglichkeiten abzuleiten:
1. Der Blutverlust insbesondere bei Schwerkranken durch häufige und z.T. mehrfache tägliche diagnostische Blutentnahmen läßt sich wesentlich senken.
2. Eine erhebliche Kostenersparnis ist erreichbar, begründet durch geringere Anschaffungs- und Entsorgungskosten der Blutentnahmeröhrchen sowie vor allem durch eine jetzt mögliche Integration verschiedener labordiagnostischer Meß- und Arbeitsplätze mit einheitlich antikoaguliertem Blut.

## Patentansprüche

1. Automatisiertes Verfahren zum Durchführen von Bluttests, **dadurch gekennzeichnet, daß** eine frisch entnommene Blutprobe mit mindestens einem Thrombin-Inhibitor, ausgewählt aus Hirudin und/oder Desulfatohirudin, versetzt wird und diese Blutprobe sowohl für die Bestimmung klinisch-chemischer Parameter als auch für die Bestimmung hämatologischer Parameter eingesetzt wird.

2. Verfahren nach Anspruch 1, **dadurch gekennzeichnet, daß** rekombinantes Hirudin und/oder Desulfatohirudin eingesetzt wird.

3. Verfahren nach einem der Ansprüche 1 bis 2, **dadurch gekennzeichnet, daß** vor der Bestimmung der klinisch-chemischen Parameter zelluläre und korpuskuläre Bestandteile aus der Blutprobe entfernt werden.

4. Verfahren nach einem der Ansprüche 1 bis 2, **dadurch gekennzeichnet, daß** das Blutbild und/oder das Differentialblutbild bestimmt wird.

5. Verfahren nach einem der Ansprüche 1 bis 2, **dadurch gekennzeichnet, daß** Blutgruppentests, Antikörpersuchtests, Coombstests oder Tests von Erythrozytenkonzentraten durchgeführt werden.

6. Verfahren nach einem der Ansprüche 1 bis 2, **dadurch gekennzeichnet, dass** eine Immunphänotypisierung mit peripherem Blut bzw. Knochenmarkblut durchgeführt wird.

7. Verfahren nach einem der Ansprüche 1 bis 6, **dadurch gekennzeichnet, daß** die frisch entnommene Blutprobe in einen Behälter gegeben wird, der den Thrombin-Inhibitor enthält.

8. Automatisiertes Verfahren zum Bestimmen von klinisch-chemischen Blutparametern, **dadurch gekennzeichnet, daß** eine frisch entnommende Blutprobe mit mindestens einem Thrombin-Inhibitor, ausgewählt aus Hirudin und Desulfatohirudin, versetzt wird und die klinisch-chemischen Parameter unter Verwendung der so erhaltenen Blutprobe bestimmt werden.

9. Verfahren nach Anspruch 8, **dadurch gekennzeichnet, daß** vor der Bestimmung der klinisch-chemischen Parameter zelluläre und korpuskuläre Bestandteile aus der Blutprobe entfernt werden.

10. Verfahren nach Anspruch 8 oder 9, **dadurch gekennzeichnet, daß** rekombinantes Hirudin und/oder Desulfatohirudin eingesetzt wird.

11. Verfahren nach einem der Ansprüche 8 bis 10, **dadurch gekennzeichnet, daß** die frisch entnommene Blutprobe in einen Behälter gegeben wird, der den Thrombin-Inhibitor enthält.

12. Verfahren nach einem der Ansprüche 8 bis 11, **dadurch gekennzeichnet, daß** die Bestimmung der klinisch-chemischen Blutparameter unter Verwendung von Blutplasma durchgeführt wird.

13. Verwendung von Blutentnahmebehältnissen, die mindestens einen Thrombin-Inhibitor, ausgewählt aus Hirudin und/oder Desulfatohirudin, enthalten, sowohl für die Bestimmung klinisch-chemischer Parameter als auch für die Bestimmung hämatologischer Parameter.

14. Verwendung von Blutentnahmebehältnissen, die mindestens einen Thrombin-Inhibitor, ausgewählt aus Hirudin und/oder Desulfatohirudin, enthalten, für die Bestimmung klinisch-chemischer Parameter.

15. Verwendung nach Anspruch 14, **dadurch gekennzeichnet, daß** der Thrombin-Inhibitor rekombinantes Hirudin und/oder Desulfatohirudin ist.

16. Verwendung nach einem der Ansprüche 12 bis 15, **dadurch gekennzeichnet, daß** die Blutentnahmebehältnisse mit mindestens einem Thrombin-Inhibitor, ausgewählt aus Hirudin und/oder Desulfatohirudin, beschichtet sind.

## Claims

1. Automated method for carrying out blood tests, **characterized in that** a freshly taken blood sample is mixed with at least one thrombin inhibitor selected from hirudin and/or desulfatohirudin, and this blood sample is employed both for determining biochemical parameters and for determining haematological parameters.

2. Method according to Claim 1, **characterized in that** recombinant hirudin and/or desulfatohirudin is employed.

3. Method according to either of Claims 1 to 2, **characterized in that** cellular and corpuscular constituents are removed from the blood sample for determination of the biochemical parameters.

4. Method according to one of Claims 1 to 2, **characterized in that** the complete blood count and/or the differential blood count is determined.

5. Method according to either of Claims 1 to 2, **characterized in that** blood group tests, antibody screening tests, Coombs tests or tests of erythrocyte concentrates are carried out.

6. Method according to either of Claims 1 to 2, **characterized in that** an immunophenotyping is carried out with peripheral blood or bone marrow blood.

7. Method according to any of Claims 1 to 6, **characterized in that** the freshly taken blood sample is put in a container containing the thrombin inhibitor.

8. Automated method for determining biochemical blood parameters, **characterized in that** a freshly taken blood sample is mixed with at least one thrombin inhibitor selected from hirudin and desulfatohirudin, and the biochemical parameters are determined using the blood sample obtained in this way.

9. Method according to Claim 8, **characterized in that** cellular and corpuscular constituents are removed from the blood sample for determination of the biochemical parameters.

10. Method according to Claim 8 or 9, **characterized in that** recombinant hirudin and/or desulfatohirudin is employed.

11. Method according to any of Claims 8 to 10, **characterized in that** the freshly taken blood sample is put in a container containing the thrombin inhibitor.

12. Method according to any of Claims 8 to 11, **characterized in that** the determination of the biochemical blood parameters is carried out using blood plasma.

13. Use of blood sampling containers which contain at least one thrombin inhibitor selected from hirudin and/or desulfatohirudin, both for determining biochemical parameters and for determining haematological parameters.

14. Use of blood sampling containers which contain at least one thrombin inhibitor selected from hirudin and/or desulfatohirudin for determining biochemical parameters.

15. Use according to Claim 14, **characterized in that** the thrombin inhibitor is recombinant hirudin and/or desulfatohirudin.

16. Use according to any of Claims 12 to 15, **characterized in that** the blood sampling containers are coated with at least one thrombin inhibitor selected from hirudin and/or desulfatohirudin.

## Revendications

1. Procédé automatisé destiné à effectuer des tests sanguins, **caractérisé en ce qu'**un échantillon sanguin récemment prélevé est mélangé avec au moins un inhibiteur de la thrombine, tel que l'hirudine et/ou la désulfato-hirudine et que cet échantillon sanguin est utilisé aussi bien pour la détermination de paramètres clinico-chimiques que pour celle de paramètres hématologiques.

2. Procédé selon la revendication 1, **caractérisé en ce que** l'hirudine et/ou la désulfato-hirudine recombinante est utilisée.

3. Procédé selon l'une quelconque des revendications 1 à 2, **caractérisé en ce que** les éléments corpusculaires et cellulaires sont extraits de l'échantillon sanguin avant la détermination des paramètres clinico-chimiques.

4. Procédé selon l'une quelconque des revendications 1 à 2, **caractérisé en ce que** la numération et formule sanguine et/ou la formule sanguine est déterminée.

5. Procédé selon l'une quelconque des revendications 1 à 2, **caractérisé en ce que** les tests de détermination du groupe sanguin, de recherche des anticorps, les épreuves à l'antiglobuline (ou réactions de Coomb) ou les tests concernant le culot d'hématies pour transfusion sont effectués.

6. Procédé selon l'une quelconque des revendications 1 à 2, **caractérisé en ce qu'**une immunophénotypisation avec du sang périphérique ou du sang de moelle osseuse est effectuée.

7. Procédé selon l'une quelconque des revendications de 1 à 6, **caractérisé en ce qu'**un échantillon sanguin récemment prélevé est placé dans un tube collecteur, qui contient l'inhibiteur de la thrombine.

8. Procédé automatisé destiné à déterminer les paramètres sanguins clinico-chimiques, **caractérisé en ce qu'**un échantillon sanguin récemment prélevé est mélangé avec au moins un inhibiteur de la thrombine, tel que l'hirudine et la désulfato-hirudine et que les paramètres clinico-chimiques sont déterminés en utilisant l'échantillon sanguin ainsi obtenu.

9. Procédé selon la revendication 8, **caractérisé en ce que** les éléments corpusculaires et cellulaires sont extraits de l'échantillon sanguin avant la détermination des paramètres clinico-chimiques.

10. Procédé selon la revendication 8 ou 9,
**caractérisé en ce que** l'hirudine et/ou la désulfato-hirudine recombinante est utilisée.

11. Procédé selon l'une quelconque des revendications 8 à 10, **caractérisé en ce que** l'échantillon sanguin récemment prélevé est placé dans un tube collecteur, qui contient l'inhibiteur de la thrombine.

12. Procédé selon l'une quelconque des revendications 8 à 11, **caractérisé en ce que** la détermination des paramètres clinico-chimiques du sang repose sur l'utilisation du plasma sanguin.

13. Utilisation d'éprouvettes à fond plat, qui contiennent au moins un inhibiteur de la thrombine, tel que l'hirudine et/ou la désulfato-hirudine et qui sont utilisées aussi bien pour la détermination des paramètres clinico-chimiques que pour celle des paramètres hématologiques.

14. Utilisation d'éprouvettes à fond plat, qui contiennent au moins un inhibiteur de la thrombine, tel que l'hirudine et/ou la désulfato-hirudine et qui sont utilisées pour la détermination des paramètres clinico-chimiques.

15. Utilisation selon la revendication 14, **caractérisée en ce que** l'inhibiteur de thrombine est l'hirudine et/ou la désulfato-hirudine recombinante.

16. Utilisation selon l'une quelconque des revendications 12 à 15, **caractérisée en ce que** les éprouvettes à fond plat sont enduites d'au moins un inhibiteur de la thrombine, tel que l'hirudine et/ou la désulfato-hirudine.
